# EUROPEAN PATENT APPLICATION

(11) **EP 1 245 237 A1**
(43) Date of publication of application: **02.10.2002**
(21) Application number: 01303094.5
(22) Date of filing: 30.03.2001
(51) Int. Cl.: A61K 38/17, A61P 17/06, A61P 17/00

(54) **Treatment of skin conditions**

(71) Applicant: University College Cardiff Consultants Limited, Cardiff CF1 3XR (GB)
(72) Inventor: Gumbleton, Mark, Cardiff, CF 14 3RH (GB); Campbell, Lee c/o Pharmaceutical Cell Biology, Cardiff University (GB)
(74) Representative: Hill, Christopher Michael

(57) **Abstract**

Provided is a use of a caveolin-1 protein, or a purified or isolated derivative or precursor thereof, in the manufacture of a medicament effective in treating keratinocyte hyperproliferation in mammalian skin, wherein the caveolin-1 protein, or purified or isolated derivative or precursor thereof, comprises a material comprising the whole or a substantial part of amino acid peptide sequence Seq. ID 1.

Also provided is a use of a peptide mimetic comprising a low molecular weight organic molecule capable of functionally mimicking the whole or a substantial part of the amino acid peptide sequence Seq. ID 1, in the manufacture of a medicament effective in treating keratinocyte hyperproliferation in mammalian skin.

Additionally provided is a use of a compound capable of stimulating the production of caveolin-1 protein, in the manufacture of a medicament effective in treating keratinocyte hyperproliferation in mammalian skin.

## Description

The present invention relates to the treatment of mammalian skin conditions, particularly psoriatic epidermal conditions, eczema, idiopathic lichen planus, or the like.

Caveolae are specialised plasmalemmal domains most prominent in terminally differentiated cell types such as smooth muscle cells, endothelial cells and alveolar type I pneumocytes. Caveolae have been implicated in a diverse range of cellular processes, for example, the regulation of signal transduction events (Review, Shaul, P.W. and Anderson, R.G.W. (1998) Am. J. Physiol. 275 L843-51). Localised in the cytoplasmic leaflet of caveolae is the integral membrane protein caveolin-1.

Caveolin-1 serves a direct regulatory role in signal transduction events acting as a 'molecular brake' for selective signalling cascades. For example, caveolin-1 functions as a negative regulator for mitogen receptor tyrosine kinases (Galbiati, F. et al. (1998) EMBO J. 17,6633-48), such as Epidermal Growth Factor (EGF) receptor. Negative regulation is achieved by caveolin-1 possessing a 'scaffolding domain' (amino acid 82 to 101 inclusive) within the N-terminal cytoplasmic region of the protein. This 'scaffolding domain' recognises and binds conserved caveolin-1 binding motifs within the catalytic domain of substrates, such as EGF receptor. The interaction between the scaffolding domain of caveolin-1 protein and the binding motif of the EGF receptor, for example, render the EGF receptor in an inactive state at the plasmalemmal.

According to the present invention there is provided caveolin-1 protein or a purified or isolated derivative or precursor thereof, for use as an inhibitor of keratinocyte hyperproliferation in mammalian skin.

We have established that caveolin-1 protein inhibits keratinocyte hyperproliferation through inhibition or negative regulation of signal transduction pathways, for example, mitogen-stimulated pathways, which, in their own right, when not regulated, lead to hyperproliferation of keratinocytes in mammalian skin.

Methods of isolating and purifying caveolae (ofwhich caveolin-1 is a main structural protein) have been previously described in US patents 5776770 and 5914127. These documents make no hint or suggestion of therapeutic targets for caveolae or caveolin-1 protein and in particular their use in the treatment of disorders of mammalian skin.

According to another embodiment of the present invention, there is provided a material comprising the whole or substantial part of the following amino acid peptide sequence Seq ID1, for use as an inhibitor of keratinocyte hyperproliferation in mammalian skin.

Seq ID1
Asp Gly Ile Trp Lys Ala Ser Phe Thr Thr Phe Thr Val Thr Lys Tyr Trp Phe Tyr Arg

This peptide sequence Seq ID1 has been identified as the scaffolding domain of caveolin-1 which interacts with a binding motif within substrates such as EGF receptor, as mentioned above.

A sequence corresponding to the above Sequence ID1 has been previously described in Engleman, J.A. et al. (J. Biol. Chem. 273, 20448-55) for inhibition of the activity of C-NEU kinase (a signalling species that may have a role in breast cancer). There is no hint or suggestion in Engleman et al. of any activity of the above sequence in treatment of skin and, in particular, there is no hint or suggestion of its use in the treatment of mammalian skin for the inhibition of keratinocyte hyperproliferation.

The peptide characterised by the amino acid sequence Seq ID1 may be isolated from naturally occurring caveolin-1 or, preferably, it may be produced by synthetic means.

According to a further embodiment of the present invention, there is provided a peptide mimetic comprising a low molecular weight organic molecule that functionally mimics the whole or substantial part of the amino acid peptide sequence Seq ID 1, for use as an inhibitor of keratinocyte hyperproliferation in mammalian skin.

According to a further embodiment of the present invention there is provided at least one compound which stimulates production of caveolin-1 protein, for use as an inhibitor of keratinocyte hyperproliferation in mammalian skin. The latter compound is preferably such as to increase the amount of natural caveolin-1 protein present in the skin.

It is a preferred feature of the present invention, that the at least one compound which stimulates production of caveolin protein activates transcription of the caveolin-1 gene, for example, it may bind and transactivate sterol binding elements.

It is another preferred feature of the present invention, that the at least one compound which stimulates production of caveolin protein promotes translation of the caveolin-1 gene, preferably through stabilising caveolin-1 mRNA.

It is further preferred feature of the present invention, that the at least one compound which stimulates production of caveolin protein stabilises caveolin-1 protein.

The present invention preferably further comprises a pharmaceutical formulation comprising any of the above compounds according to the present invention, together with a pharmaceutically acceptable carrier, diluent or excipient therefor, for use as an inhibitor of keratinocyte hyperproliferation in mammalian skin.

According to the present invention, the preferred route of administration of the formulation is topical. The formulation may be in the form of a gel, foam, salve, emollient, cream or ointment which may be applied to the affected skin.

Alternatively, a pharmaceutical formulation according to the present invention may be in a form suitable for oral administration.

Hyperproliferation and abnormal keratinocyte differentiation lead to several disorders of the skin, such as, for example, psoriasis. Psoriasis is a chronic hyperproliferative skin disorder characterised by itchy flaky skin. It is thought that 1 to 2% of the human population suffers with psoriasis.

A number of therapies exist for the treatment of hyperproliferative skin disorders. Current therapies include the application of antimetabolites (such as methotrexate), corticosteriods (such as hydrocortisone and triamcinolone creams or injections), psoralens and photochemotherapy, vitamin D analogues and retinoids. However, these therapies only offer temporary relief and are all associated with unwanted side effects such as hypercalcemia, adrenal insufficiency, hypertension and predisposition of malignant disease.

The compound or pharmaceutical formulation according to the present invention is preferably suitable for the treatment of hyperproliferative skin disorders, preferably human hyperproliferative skin disorders.

The human skin disorder to be treated using a compound or pharmaceutical formulation according to the present invention, is preferably psoriasis. Psoriasis epidermal conditions may include, but are not limited to, exfoliative psoriatic dermatitis, pustular psoriasis, and guttatte variant psoriasis.

Other skin conditions, such as, for example, eczema may be treated using a compound or pharmaceutical formulation according to the present invention. Eczema is an inflammatory condition of the skin which may have some associated keratinocyte hyperproliferation.

According to a further embodiment of the present invention, there is provided at least one amino acid peptide sequence comprising the whole or substantial part of a protein binding motif which is capable of interacting with caveolin-1 protein scaffolding domain, for use as a stimulator of keratinocyte proliferation in mammalian skin.

Short peptide sequences comprising protein binding motifs which interact directly with caveolin-1 scaffolding domain have been previously described in Couet, J. et al.(1997) J. Biol. Chem. 272, 6525-33. The peptide sequences identified in Couet et al. comprise the following spacing:

ΦXΦXXXXΦ;
ΦXXXXΦXXΦ; or
ΦXΦXXXXΦXXΦ.

Where Φ is any of aromatic amino acids Tryptophan, Phenyalanine or Tyrosine, and X is any amino acid. These peptide sequences competitively inhibit the interaction of caveolin-scaffolding domain with caveolin-1 binding motifs within the catalytic domain of substrates, such as G_{12α} protein. There is no hint or suggestion in Couet *et al*. of any activity of the above peptide sequences in treatment of skin and, in particular, there is no hint or suggestion of its use in the treatment of mammalian skin for stimulation of keratinocyte proliferation.

In certain skin conditions, such as, for example, idiopathic lichen planus, there is hypoproliferation of skin keratinocytes. Idiopathic lichen planus is a condition where skin keratinocyte hypoproliferation, results in apoptosis and a breakdown in the basal layer of the epidermis.

The at least one protein binding motif amino acid peptide sequence according to the present invention, may inhibit the ability of caveolin-1 to function as a negative regulator. Without negative regulation of caveolin-1, many of the signal transduction pathways, for example, mitogen-stimulated pathways, may not be regulated, thereby resulting in keratinocyte hyperproliferation. The at least one protein binding motif amino acid peptide sequence is therefore preferably used in skin conditions where there is hypoproliferation of skin keratinocytes, preferably the skin condition idiopathic lichen planus. Further, treatments that reverse keratinocyte hypoproliferation would have a use in wound healing processes and within the cosmetic area, particularly in regard to preventing or reversing skin wrinkling caused by the ageing process.

According to a further embodiment of the present invention, there is provided at least one peptide mimetic comprising a low molecular weight organic molecule that functionally mimics an amino acid peptide sequence comprising the whole or substantial part of a protein binding motif which is capable of interacting with caveolin-1 protein scaffolding domain, for use as a stimulator of keratinocyte proliferation in mammalian skin.

The present invention further comprises a pharmaceutical formulation comprising at least one amino acid peptide sequence comprising the whole or substantial part of a protein binding motif which is capable of interacting with caveolin-1 protein scaffolding domain (and/or at least one peptide mimic which functionally mimics said at least one amino acid peptide sequence) according to the present invention, together with a pharmaceutically acceptable carrier, diluent or excipient therefor, for use as a stimulator of keratinocyte proliferation in mammalian skin.

According to the present invention, the preferred route of administration of the formulation is topical. The formulation may be in the form of a gel, foam, salve, emollient, cream or ointment which may be applied to the affected skin.

Alternatively, a pharmaceutical formulation according to the invention may be in a form suitable for oral administration.

### Examples

### Immunocytochemical methods of analysis of skin sections

Archival biopsied human skin specimens were obtained from the Department of Pathology, University of Wales, College of Medicine. Specimens were derived from subjects who have a clinical diagnosis of psoriasis and normal subjects that have undergone routine biopsies for comparative purposes. The term 'normal' is defined to include its generally accepted meaning and includes human dermal tissue where the histological features of psoriasis such as hyperkeratosis, parakeratosis, dermal capillary angiogenesis, marked acanthosis and dermal infiltration of inflammatory cells are absent. Resected tissues have been fixed in 10 % formalin washed in double distilled water (ddH₂O, 2X15min) and dehydrated in an ethanol/xylene gradient and embedded in paraffin wax using a Shanndon (Shanndon, Cheshire, UK) automated tissue processor. Paraffin wax sections (5-10µm) were cut from the resulting blocks and mounted on superfrost microscope slides (Shanndon) in preparation for immunostaining. Six affected individuals and six normal controls were examined.

Following removal of the paraffin any residual endogenous hydrogen peroxidase activity within the tissue sections was quenched by a 15min pre-treatment with a methanol/hydrogen peroxide mix (47.2ml methanol/0.8ml hydrogen peroxide). The sections were then washed in running tap water for a further 5 min. They were then placed in a coplin jar containing opimax wash buffer (PBS with surfactant pH 7.6) (BioGenex, San Ramon, CA, USA) and equilibrated for 10 min. The rehydrated sections were placed in a humidified chamber and sections covered in 100µl of polyclonal anti-caveolin-1 antibody at a dilution of 1:20 made from the 0.25µg/ml antibody stock solution (host-rabbit; Transduction Labs, USA CODE C13630). The diluent used was 0.6% BSA in optimax wash buffer. Further sections were included to act as controls in which the primary antibody was omitted or substituted with an appropriate non-immune serum (normal rabbit serum obtained from DAKO, U.K.). The primary antibody was incubated overnight at 4EC. The next day the sections were then washed 4X1min in optimax wash buffer and covered for 1hr with 100µl swine anti-rabbit IgG antibody conjugated to HRP (DAKO, U.K.) diluted in optimax wash buffer containing 0.6% BSA. Incubation with the secondary antibody (1hr) was undertaken in an humidified chamber at room temperature. The sections were then washed 4 X 1 min in optimax wash buffer and placed into a coplin jar containing fresh wash buffer. The wash buffer was then removed and 200mls of Diaminobenzidine dihydrochloride (DAB) (working solution 0.5g/l) added for 5min for development of the immunostain. Following this the slides were washed in running tap water and counterstained with haematoxylin. The sections were then dehydrated through an alcohol/xylene gradient and mounted in DPX mountant before analysis by bright field light microscopy.

### Skin biopsies derived from non-psoriatic subjects

The following are results of immunohistochemical staining on normal and psoriatic skin sections.

Histological samples were scored as follows:
- +++: Intense caveolin label within the epidermis
- ++: Moderate caveolin label within the epidermis
- +: Weak caveolin label within the epidermis
- +/-: Weak barely detectable caveolin label within the epidermis
- -: No signal detectable

### Example 1

Skin biopsy was taken from a male aged 75 years (Lab/No S,98.0012802.B) where the histological features of the dermis and epidermis exhibited all the features of normal morphology. Specifically none of the characteristics of the psoriatic phenotype (i.e. hyperproliferative elongated retes, dermal angiogenesis, parakeratosis, increased number of mitotic keratinocytes, pustules of Kogi and presence of neutrophils in the dermal/epidermal regions. Intense caveolin immunostain was present in all layers of the epidermis but was more pronounced in the proliferative cells of the basal layer and cells of the more differentiated upper granular layers. Endothelium in the lower regions of the dermis stained intensely for caveolin as expected. Additionally nerve tissue which is generally regarded as caveolin negative stained very weakly or not at all. These two features act as appropriate positive and negative internal controls respectively and authenticate the distribution and extent of the specific caveolin immunostain within normal epidermis.
Score out of (3) +++

### Example 2

Skin biopsy was taken from a male aged 82yrs (Lab No/Spec S,98.0011654.H). Histological features and the pattern and intensity of caveolin immunostain was as in example 1. Score +++

### Example 3

Skin biopsy was taken from a female aged 25yrs (Lab No/Spec S,98.0013052.N). Histological features and the pattern and intensity of caveolin immunostain was as in Example 1.
Score +++

### Example 4

Skin biopsy was taken from a male aged 21yrs (Lab No/Spec S,98.00131098.Z). Histological features and the pattern and intensity of caveolin immunostain was as in Example 1.
Score +++

### Example 5

Skin biopsy was taken from a female aged 25yrs (Lab No/Spec S,98.0013553.S). Histological features was identical and the pattern and intensity of caveolin immunostain similar as that described for Example 1.
Score ++

### Example 6

Skin biopsy was taken from a female aged 61yrs (Lab No/Spec S,98.0007768.K) Histological features was identical and the pattern and intensity of caveolin immunostain similar as that described for Example 1.
Score ++

### Psoriatic cases

### Example 1

Skin biopsy was taken from a male aged 42 yrs (Lab No/Spec S,98.0013424.A) with a clinical diagnosis of psoriasis. Histological features showed all the major hallmarks of the gross psoriatic phenotype such as parakeratosis, presence of elongated epidermal retes, marked dermal angiogenesis, infiltration of neutrophils within the dermis and increased number of mitotic keratinocytes within the hyperproliferative regions of the epidemis. Immunostain against caveolin-1 protein was predominantly localised to the more differentiated layers of the epidermis (spinious/granular layers). Label was greatly diminished or totally absent in the elongated hyperproliferative epidermal retes, except in individual cells undergoing mitosis where staining against caveolin was intense. Endothelial vessels in the lower regions of the uninvolved dermis stained intensely for caveolin thus authenticating the pattern and distribution of caveolin immunolabel in the involved regions of the specimen.
Score +

### Example 2

Skin biopsy was taken from a male aged 64yrs (Lab No/Spec S,98.0004623.P) with a clinical diagnosis of psoriasis. Hyperproliferative elongated retes were present that were seen to coalesce in regions. Additionally, pustules of Kogi, a definitive histological feature of psoriasis, were evident along with marked dermal angiogenesis and inflammatory infiltrate Immunostain against caveolin-1 protein was absent from the elongated retes except in individual cells undergoing mitosis where once again caveolin stain was intense. In this particular case specific label was just perceptible in the more differentiated regions of the epidermis.
Score -/+

### Example 3

Skin biopsy was taken from a male aged 18yrs (Lab No/Spec S,98.0013843.W) with a clinical diagnosis of pustular psoriasis. Elongated retes are absent but evidence of hyperproliferation and spongiosis are present. Immunostain specific for caveolin-1 is greatly diminished and once again barely perceptible in higher levels of the epidermis. Cells undergoing the process of mitosis are numerous in the regions suggestive of hyperproliferation and stain intensely for caveolin.
Score -/+

### Example 4

Skin biopsy was taken from a male aged 52 years (Lab No/Spec S,98.0013424.A) with a clinical diagnosis of psoriasis. Hyperproliferative coalescent elongated retes were present along with pustules of Kogi. Evidence of parakeratosis is exhibited and also angiogenesis in the regions of the dermis in close proximity of the involved epidermis. Numerous mitotic keratinocytes are present in the hyperproliferative regions of the epidermis and stained positive for caveolin. Caveolin immunostain is greatly diminished overall with very little present in all levels of the epidermis (granular, spinious and basal), a single layer of cells in the outermost level of the basal layer stained moderately for caveolin. Endothelial vessels in the lower regions of the uninvolved dermis stained intensely for caveolin and which serves as an appropriate internal control thus authenticating the immunolabel observed in this particular section.
Score -/+

### Example 5

Skin biopsy was taken from a female aged 40yrs (Lab No/Spec S,98.0002243.L) with a clinical diagnosis of psoriasis. All the major hallmarks of the gross psoriatic phenotype and present and includes hyperproliferative elongated retes, pustules of Kogi, parakeratosis, inflammatory infiltrate and an increase in the number of mitotic bodies in the proliferative regions of the epidermis. Immunostain for caveolin-1 is diminished in all regions of the epidermis when compared to normal controls and just perceptible overall.
Score +

### Example 6

Skin biopsy was taken from a female aged 27yrs (Lab No/Spec S,96.0008147.X) with a clinical diagnosis of psoriasis. Hyperproliferative elongated retes were present along with pronounced parakeratosis and marked dermal inflammatory infiltrate. As for psoriatic examples 1+2 specific immunosignal for caveolin-1 was diminished in the elongated hyperproliferative retes but was detectable in the more differentiated upper regions of the epidermis.
Score +

For all the above normal donors and psoriatic patients examined controls slides were run in parallel where the primary antibody has been omitted or replaced with non-immune serum. In all cases no immunostain was observed thus authenticating the results obtained from the experimental sections.

## Claims

1. Use of a caveolin-1 protein, or a purified or isolated derivative or precursor thereof, in the manufacture of a medicament effective in treating keratinocyte hyperproliferation in mammalian skin, wherein the caveolin-1 protein, or purified or isolated derivative or precursor thereof, comprises a material comprising the whole or a substantial part of amino acid peptide sequence Seq. ID 1.

2. Use of a peptide mimetic comprising a low molecular weight organic molecule capable of functionally mimicking the whole or a substantial part of the amino acid peptide sequence Seq. ID 1, in the manufacture of a medicament effective in treating keratinocyte hyperproliferation in mammalian skin.

3. Use of a compound capable of stimulating the production of caveolin-1 protein, in the manufacture of a medicament effective in treating keratinocyte hyperproliferation in mammalian skin.

4. Use according to any preceding claim, wherein the medicament is effective in the treatment of psoriasis.

5. A pharmaceutical composition comprising a protein, peptide, compound or derivative as defined in any of claims 1-3, which composition further comprises a pharmaceutically acceptable carrier, diluent or excipient.
